# EUROPEAN PATENT APPLICATION

(11) **EP 4 420 526 A1**
(43) Date of publication of application: **28.08.2024**
(21) Application number: 24156645.4
(22) Date of filing: 08.02.2024
(51) Int. Cl.: A23L 33/105, A61K 31/37, A61K 36/752

(54) **COMPOSITION FOR PREVENTING OR TREATING MUSCULAR DISEASE CONTAINING PONCIRI FRUCTUS EXTRACT**

(30) Priority: 13.02.2023 KR 20230018844; 03.07.2023 KR 20230085747
(71) Applicant: Korea Institute of Science and Technology, Seoul 02792 (KR)
(72) Inventor: KIM, Myung Suk, 25451 Gangwon-do (KR); JUNG, Sang Hoon, 25451 Gangwon-do (KR); KO, Hye Jin, 25451 Gangwon-do (KR); TAM, Thi Le, 25451 Gangwon-do (KR); NGUYEN, Bao Ngo, 25451 Gangwon-do (KR); LEE, Wook Bin, 25451 Gangwon-do (KR)
(74) Representative: Handley, Matthew Edward

(57) **Abstract**

The present invention relates to a composition for the prevention or treatment of a muscle disease, comprising an extract of Ponciri Fructus. The extract of the present invention has the effect of increasing the expression of genes related to myotube cell differentiation and/or athletic performance, increasing muscle weight, and/or improving athletic performance.

## Description

### [Technical Field]

This application claims benefit of priority based on Korean Patent Application No. 10-2023-0018844 filed on February 13, 2023 and No. 10-2023-0085747 filed on July 3, 2023, the disclosures of which are incorporated herein by reference in their entireties.

The present invention relates to a composition for the prevention or treatment of a muscle disease, comprising Ponciri Fructus extract.

### [Background Art]

Muscle is broadly categorized into skeletal muscle, cardiac muscle, and visceral muscle, with skeletal muscle being the most abundant tissue in the human body, accounting for 40-45% of body weight. Skeletal muscles attach to bones through tendons and are responsible for generating bone movement or force. A muscle is made up of many myofibrils, which are made up of many fibrils of actin and myosin. As actin and myosin move over each other, they shorten or lengthen the length of the muscle, causing the overall muscle to contract and relax. An increase in the size of the myofibrils means an increase in the thickness of the muscle fibers, resulting in muscle gain.

A muscle disease is characterized by skeletal muscle weakness that gradually leads to impaired gait and mobility, difficulty with activities of daily living (ADLs), and inability to live independently. In addition, it can lead to cardiopulmonary dysfunction and other complications, so it is important to understand the characteristics of each muscle disease.

South Korea entered an aging society in 2000, with the elderly population accounting for 7.2% of the total population, and is predicted to enter an ultra-elderly society (over 20%) by 2050 (2013 Elderly Statistics, Statistics Korea). As a person's muscle mass decreases with age (by 10-15% between ages of 50 and 70, and by more than 30% between ages of 70 and 80), muscle strength and muscle function also decrease, a condition called sarcopenia. Sarcopenia is a major cause of impaired mobility and gait, limiting independent living in older adults. In addition, sarcopenia reduces ultra-low metabolic rate, which increases insulin resistance, promotes the development of type 2 diabetes, and increases the risk of hypertension and cardiovascular disease by 3-5 times. Currently, there are no drugs approved for the treatment of sarcopenia, and drug repositioning technologies are under development to reposition myostatin inhibitors or other FDA-approved drugs for sarcopenia.

On the other hand, Ponciri Fructus is the dried immature fruit of the tangerine tree (*Poncirus trifoliata* Rafinesque) of *Rutaceae* family, and is distinguished from Aurantii Fructus. Ponciri Fructus has been used to treat conditions such as flatulence, constipation, allergies, and inflammation. Recent studies on its biological activities have reported anticancer, antibacterial, antiviral, inhibition of melanin synthesis, uterine contraction, allergy, and hypersensitivity reactions. It has also been reported that the coumarin component of Ponciri Fructus inhibits platelet aggregation. In the Dongui-bogam (Principles and Practice of Eastern Medicine), Ponciri Fructus was used to cure illnesses of the mind and stomach by descending medicinal energy, and Aurantii Fructus was used to cure illnesses of the skin and ribcage by ascending medicinal energy, the opposite of Ponciri Fructus.

The present inventors have endeavored to develop natural products with preventive or therapeutic effects on a muscle disease and have experimentally confirmed that the extract of Ponciri Fructus can significantly increase the expression of genes related to myotube cell differentiation and/or athletic performance.

### [Disclosure]

### [Technical Problem]

The object of the present invention is to provide a pharmaceutical composition for preventing or treating a muscle disease comprising Ponciri Fructus extract, a fraction thereof or auraptene as an active ingredient.

In addition, the object of the present invention is to provide a food composition for preventing or ameliorating (or improving) muscle disease, and/or for increasing muscle weight, and/or improving athletic performance comprising Ponciri Fructus extract, a fraction thereof or auraptene as an active ingredient.

In addition, the object of the present invention is to provide a quasi-drug composition for preventing or ameliorating (or improving) a muscle disease, and/or for increasing muscle weight, and/or improving athletic performance comprising Ponciri Fructus extract, a fraction thereof or auraptene as an active ingredient.

In addition, the object of the present invention is to provide a method for preventing or treating a muscle disease comprising the step of administering or ingesting a composition containing Ponciri Fructus extract, a fraction thereof or auraptene an active ingredient to a subject.

In addition, the present invention provides a use of Ponciri Fructus extract, a fraction thereof or auraptene for the manufacture of a medicament for preventing or treating a muscle disease.

However, the technical scopes of the present invention are not limited to those mentioned above, and other scopes not mentioned will be apparent to those skilled in the art from the following description.

### [Technical Solution]

The present invention provides a food composition for preventing or ameliorating (or improving) a muscle disease, and/or for increasing muscle weight, and/or improving athletic performance comprising Ponciri Fructus extract, a fraction thereof or auraptene as an active ingredient.

The present invention also provides a quasi-drug composition for preventing or ameliorating (or improving) a muscle disease, and/or for increasing muscle weight, and/or improving athletic performance comprising Ponciri Fructus extract, a fraction thereof or auraptene as an active ingredient.

The present invention also provides a pharmaceutical composition for preventing or treating a muscle disease, comprising Ponciri Fructus extract, a fraction thereof or auraptene as an active ingredient.

The present invention also provides a method for preventing or treating a muscle disease comprising the step of administering or ingesting a composition containing Ponciri Fructus extract, a fraction thereof or auraptene an active ingredient to a subject in need thereof. The composition may be a food composition, a health functional food (or nutraceutical) composition, a pharmaceutical composition, or a quasi-drug composition.

The present invention also provides a use of Ponciri Fructus extract, a fraction thereof or auraptene for the manufacture of a medicament for preventing or treating a muscle disease.

The muscle disease may be one or more diseases selected from the group consisting of sarcopenia, muscular atrophy, myasthenia, muscular dystrophy, sarcopenia, hypotonia, and muscular weakness, muscular dystrophy, atony, myoatrophic lateral sclerosis, and inflammatory myopathy.

The composition may increase the expression of one or more genes selected from the family of markers of myotube cell differentiation comprising the genes *Myf5, Myf6, Myod,* and/or *Myog.*

The composition may increase the expression of *Ppard* and/or *Pgc1a* genes, which are markers of athletic performance.

The composition may be extracted with a solvent selected from the group consisting of water, organic solvents, and mixtures thereof.

The organic solvent may be one or more solvents selected from the group consisting of lower alcohols of carbon number 1 to 6, hexane, acetone, ethyl acetate, chloroform, and diethyl ether.

### [Advantageous Effects]

The present composition including Ponciri Fructus extract, a fraction thereof or auraptene has the effect of increasing the expression of genes related to myotube cell differentiation and/or athletic performance, increasing muscle weight, and/or improving athletic performance. Furthermore, the present composition including Ponciri Fructus extract, a fraction thereof or auraptene can be applied to various products such as a pharmaceutical composition, a food composition, and/or a quasi-drug composition for the prevention, treatment, or amelioration of a muscle disease.

### [Description of Drawings]

FIG. 1 shows the effects of Ponciri Fructus extract on myotube cell viability (Con: control; Dex: Dexamethasone; PT 20: Ponciri Fructus extract 20 ug/ml; PT 10: Ponciri Fructus extract 10 ug/ml; PT 5: Ponciri Fructus extract 5 ug/ml; *, p < 0.05; ***, p < 0.001).
FIG. 2 shows the effects of Ponciri Fructus extract in enhancing the expression of genes related to myotube cell differentiation (Con: Control, Dex: Dexamethasone, PT 20: Ponciri Fructus extract 20 ug/ml, PT 10: Ponciri Fructus extract 10 ug/ml, PT 5: Ponciri Fructus extract 5 ug/ml).
FIG. 3 shows the effects of Ponciri Fructus extract in enhancing the expression of performance-related genes (Con: Control, Dex: Dexamethasone, PT 20: 20 ug/ml of Ponciri Fructus extract, PT 10: 10 ug/ml of Ponciri Fructus extract, PT 5: 5 ug/ml of Ponciri Fructus extract).
FIGS. 4 and 5 show the effects of Ponciri Fructus extract in increasing muscle force (Con: Control, Dex: Dexamethasone, Oxy: Oxymetholone, PT 100: 100 mg/kg Ponciri Fructus extract, PT 50: 50 mg/kg Ponciri Fructus extract, PT 10: 10 mg/kg Ponciri Fructus extract; ** p < 0.01; ***, p < 0.001).
FIG. 6 shows the effects of Ponciri Fructus extract on weight gain of quadricep muscle (Con: Control, Dex: Dexamethasone, Oxy: Oxymetholone, PT 100: 100 mg/kg Ponciri Fructus extract, PT 50: 50 mg/kg Ponciri Fructus extract, PT 10: 10 mg/kg Ponciri Fructus extract; ** p < 0.01; ***, p < 0.001).
FIG. 7 shows the effects of Ponciri Fructus extract on weight gain of gastrocnemius muscle (Con: Control, Dex: Dexamethasone, Oxy: Oxymetholone, PT 100: 100 mg/kg of Ponciri Fructus extract, PT 50: 50 mg/kg of Ponciri Fructus extract, PT 10: 10 mg/kg of Ponciri Fructus extract; * p < 0.05).
FIG. 8 shows the effects of Ponciri Fructus extract on weight gain of tibialis muscle (Con: control, Dex: Dexamethasone, Oxy: Oxymetholone, PT 100: 100 mg/kg Ponciri Fructus extract, PT 50: 50 mg/kg Ponciri Fructus extract, PT 10: 10 mg/kg Ponciri Fructus extract; *, p < 0.05; ** p < 0.01).
FIGS. 9 to 1 show the effects of Ponciri Fructus extract in improving athletic performance (Con: Control, Dex: Dexamethasone, Oxy: Oxymetholone, PT 100: 100 mg/kg Ponciri Fructus extract, PT 50: 50 mg/kg Ponciri Fructus extract, PT 10: 10 mg/kg Ponciri Fructus extract; *, p < 0.05).
FIG. 12 shows the effects of the fractions of Ponciri Fructus extract on myotube cell viability (*, p < 0.05; ** p < 0.01; ***, p < 0.001).
FIG. 13 shows the effects of Ponciri Fructus-derived compounds on myotube cell viability (*, p < 0.05; ** p < 0.01; ***, p < 0.001).
FIG. 14 shows the effects of auraptene on myotube cell viability (Con: Control, Dex: Dexamethasone, AUR 20: auraptene 20 ug/ml, AUR 10: auraptene 10 ug/ml, AUR 5: auraptene 5 ug/ml; *, p < 0.05; ** p < 0.01; ***, p < 0.001).
FIG. 15 shows the effects of auraptene on myofibrillogenic activity (Con: Control, Dex: Dexamethasone, AUR 20: auraptene 20 ug/ml, AUR 10: auraptene 10 ug/ml, AUR 5: auraptene 5 ug/ml; *, p < 0.05; ** p < 0.01).
FIG. 16 shows the effects of auraptene in promoting muscle differentiation-related gene expression (Con: control, Dex: dexamethasone, AUR 20: auraptene 20 ug/ml, AUR 10: auraptene 10 ug/ml, AUR 5: auraptene 5 ug/ml; *, p < 0.05; ** p < 0.01).
FIG. 17 shows the effects of auraptene in increasing the expression of athletic performance-related genes (Con: control, Dex: dexamethasone, AUR 20: auraptene 20 ug/ml, AUR 10: auraptene 10 ug/ml, AUR 5: auraptene 5 ug/ml *, p < 0.05; ** p < 0.01).
FIG. 18 shows the effects of auraptene in improving oxidative stress and mitochondrial function (Con: Control, Dex: Dexamethasone, AUR 20: auraptene 20 ug/ml, AUR 10: auraptene 10 ug/ml, AUR 5: auraptene 5 ug/ml *, p < 0.05; ** p < 0.01; p < 0.001).
FIG. 19 shows the effects of auraptene on improving muscle force (Con: control, Dex: dexamethasone, AUR 50: auraptene 50 mg/kg, AUR 10: auraptene 10 mg/kg; *, p < 0.05; ** p < 0.01; p < 0.001).
FIG. 20 shows the effect of auraptene on increasing muscle weight (thigh muscle weight, longissimus dorsi muscle weight, calf muscle weight, and sole muscle weight) (Con: control, Dex: dexamethasone, AUR 50: auraptene 50 mg/kg, AUR 10: auraptene 10 mg/kg; *, p < 0.05; ** p < 0.01; p < 0.001).

### [Modes of the Invention]

The inventors completed the present invention by experimentally confirming that an extract of Ponciri Fructus, a natural product with few or no side effects, is effective in increasing the expression of genes related to myotube cell differentiation and/or athletic performance, increasing muscle weight, and/or improving athletic performance.

The present invention will now be described in detail.

The present invention provides a composition for the prevention, amelioration or treatment of a muscle disease comprising Ponciri Fructus extract or a fraction thereof as an active ingredient.

The muscle disease may be one or more diseases selected from the group consisting of sarcopenia, muscular atrophy, myasthenia, muscular dystrophy, sarcopenia, hypotonia, and muscular weakness, muscular dystrophy, atony, myoatrophic lateral sclerosis, and inflammatory myopathy.

The composition may increase the expression of one or more genes selected from the family of markers of myotube cell differentiation comprising the genes *Myf5, Myf6, Myod,* and/or *Myog.*

The composition may increase the expression of *Ppard* and/or *Pgc1a* genes, which are markers of athletic performance.

The composition may be a food composition, a health functional food (or nutraceutical) composition, a pharmaceutical composition, or a quasi-drug composition.

As used herein, "preventing" means any act of delaying the onset of a muscle disease by administration of a composition of the present invention, and "treating" and "ameliorating" means any act of ameliorating or beneficially altering the symptoms of a muscle disease by administration of a composition of the present invention.

Ponciri Fructus extract (also refer to "extract of Ponciri Fructus") can be extracted using polar solvents and/or non-polar solvents.

The polar solvent may comprise one or more selected from the group consisting of (i) water, (ii) alcohol (preferably, methanol, ethanol, propanol, butanol, normal-propanol, isopropanol, normal-butanol, 1-pentanol, 2-butoxyethanol or ethylene glycol), (iii) acetic acid, (iv) dimethyl-formamide (DMFO), and (v) dimethyl sulfoxide (DMSO). The non-polar solvents may include acetone, acetonitrile, ethyl acetate, methyl acetate, fluoroalkanes, pentane, hexane, 2,2,4-trimethylpentane, decane, cyclohexane, cyclopentane, diisobutylene, 1-pentene, 1-chlorobutane, 1-chloropentane, o-xylene, diisopropyl ether, 2-chloropropane, toluene, 1-chloropropane, chlorobenzene, benzene, diethyl ether, diethyl sulfide, chloroform, dichloromethane, 1,2-dichloroethane, aniline, diethylamine, ether, carbon tetrachloride, and THF.

More specifically, the present extract may be extracted using water, an organic solvent, or a mixture thereof as a solvent. According to certain embodiments of the invention, the extract of the invention may be obtained by treating Ponciri Fructus with ethanol. More preferably, the extract may be obtained by treating Ponciri Fructus with 70-100% (v/v) ethanol.

The organic solvent may comprise one or more solvents selected from the group consisting of lower alcohols having a carbon number of 1 to 6 (methanol, ethanol, propanol, butanol, and the like), hexane, acetone, ethyl acetate, chloroform, and diethyl ether.

Furthermore, the present Ponciri Fructus extract may be prepared as a fraction according to methods conventionally used in the art, and the solvent used to fractionate Ponciri Fructus extract may be one or more selected from the group consisting of, but not limited to, water, lower alcohols of C₁ to C₄, n-hexane, ethyl acetate, acetone, acetonitrile, butyl acetate, 1,3-butylene glycol, methylene chloride, and mixtures thereof. However, methylene chloride is preferably used.

As used herein, the term "extract" has the meaning conventionally understood in the art as a crude extract as described above, but broadly includes fractions that are further fractionated from the extract, i.e., the extract is not only obtained using the extraction solvent described above, but also includes fractions obtained by applying additional purification processes. For example, fractions obtained by passing the extract through an ultrafiltration membrane having a certain molecular weight cut-off value, separation by various chromatographies (designed for separation based on size, charge, hydrophobicity or affinity), fractions obtained by various further purification methods, etc. are also included in the extracts of the present invention.

The extracts utilized in the present invention may be prepared in a powdered state by reduced pressure distillation and further processes such as freeze drying or spray drying.

Specifically, the present extract may be extracted by adding water, an organic solvent, or a mixture thereof to Ponciri Fructus in an amount of from 5 to 50 times the weight of Ponciri Fructus, more preferably from 10 to 30 times the weight of Ponciri Fructus, but not limited to. The extraction temperature may be 10 °C to 150 °C, more preferably 15 °C to 120 °C, but not limited to. The extraction time is preferably from 1 hour to 20 hours, more preferably from 2 hours to 8 hours, but not limited to. The extraction method may be, but is not limited to, cold steeping, ultrasonic extraction, or reflux cooling extraction. The number of extraction cycles is preferably from 1 to 5, and more preferably from 2 to 3 repeated extractions. Furthermore, the extract may be diluted, concentrated, or purified and dried after dilution or concentration.

The Ponciri Fructus extract and/or fraction(s) thereof according to the present invention may comprise one or more compounds selected from the group consisting of compounds represented by the formula 1 to 13 (see Table 1 below). Preferably, it may comprise one or more compounds selected from the group consisting of compounds represented by Formula 2, Formula 3, Formula 9, and Formula 12. More preferably, it may comprise a compound represented by Formula 2.

As used herein, the term "comprising as an active ingredient" means comprising an amount sufficient to achieve the efficacy or activity of Ponciri Fructus extract. The present invention is a composition derived from Ponciri Fructus, and the upper limit of amount of Ponciri Fructus extract included in the composition of the present invention may be selected and practiced by those skilled in the art within a suitable range.

### A pharmaceutical composition for the prevention or treatment of a muscle disease, comprising Ponciri Fructus extract as an active ingredient

The composition of the present invention can be formulated as a pharmaceutical composition.

When the composition of the present invention may be formulated as a pharmaceutical composition, the pharmaceutical composition of the present invention may comprise a pharmaceutically acceptable carrier.

According to a preferred embodiment of the present invention, the composition of the present invention may be a pharmaceutical composition comprising (a) a pharmaceutically effective amount of Ponciri Fructus extract of the present invention as described above; and (b) a pharmaceutically acceptable carrier. As used herein, the term "pharmaceutically effective amount" refers to an amount sufficient to achieve the efficacy or activity of above-described Ponciri Fructus extract.

Pharmaceutically acceptable carriers are those customarily used in formulation: lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil. In addition to above components, the pharmaceutical composition of the present invention may further comprise lubricants, wetting agents, sweeteners, flavoring agents, emulsifiers, suspending agents, preservatives, and the like. Suitable pharmaceutically acceptable carriers and formulations are described in detail in Remington's Pharmaceutical Sciences (19th ed., 1995).

The pharmaceutical composition of the present invention can be administered orally or parenterally.

Suitable dosages of the pharmaceutical composition of the invention may be prescribed in a variety of ways, depending on factors such as method of formulation, mode of administration, patient's age, weight, sex, medical condition, food, time of administration, route of administration, rate of excretion, and response sensitivity. Typical dosages of active ingredients in the pharmaceutical composition of the present invention are in the range of 0.001-100 mg/kg/day, preferably 0.01-35 mg/kg/day for adults. The dose may be administered once a day or may be divided into several doses. However, above dosages do not limit the scope of the present invention.

The pharmaceutical composition of the present invention may be prepared in unit dose form or in multi-dose containers by formulation with pharmaceutically acceptable carriers and/or excipients, according to methods readily practiced by a person of ordinary skill in the art to which the invention belongs. The formulation may be in the form of a solution, suspension, syrup or emulsion in an oil or aqueous medium, or in the form of an excipient, acid, powder, granule, tablet or capsule, and may further comprise a dispersant or stabilizing agent.

### A quasi-drug composition for the prevention or amelioration of a muscle disease, comprising an extract of Ponciri Fructus as an active ingredient

The composition of the present invention can be provided as quasi-drug composition.

The Ponciri Fructus extract may be added as is, or may be used in combination with other quasi-drug ingredients, and may be used as appropriate according to conventional methods. Amount of mixing of active ingredients may be suitably determined according to the intended use (preventive, health or therapeutic treatment). The quasi-drug composition may be used in the preparation of, but are not limited to, topical preparations, patches, ointments, and the like.

### A food composition for the prevention or amelioration of a muscle disease, comprising Ponciri Fructus extract as an active ingredient

The composition of the present invention can be provided as a food composition or dietary supplement composition. When the composition for the prevention, amelioration or treatment of a muscle disease comprising Ponciri Fructus extract of the present invention as an active ingredient may be prepared as food a composition, they include not only Ponciri Fructus extract as an active ingredient, but also ingredients that are customarily added in the manufacture of food products, for example, proteins, carbohydrates, fats, nutrients, seasonings and flavoring agents. Examples of the carbohydrates described above are monosaccharides, e.g., glucose, fructose, etc.; disaccharides, e.g., maltose, sucrose, oligosaccharides, etc.; and polysaccharides, e.g., dextrins, cyclodextrins, etc., as well as conventional sugars and sugar alcohols such as xylitol, sorbitol, erythritol, etc. As flavoring agents, natural flavoring agents [such as thaumatin, stevia extract (e.g., rebaudioside A, glycyrrhizin, etc.)] and synthetic flavoring agents (such as saccharin, aspartame, etc.) can be used. For example, if the food composition of the present invention is prepared as a beverage, it may additionally contain citric acid, liquid sugar, sugar, glucose, acetic acid, malic acid, nectar, caterpillar extract, jujube extract, licorice extract, etc. in addition to the natural product extract of the present invention.

The formulation of the food composition or dietary supplement composition can be in the form of an acid, granule, pill, tablet, or capsule, as well as in the form of a conventional food or beverage.

Examples of foods to which the substances may be added include, without limitation, meat, sausages, bread, chocolate, candy, snacks, confectionery, pizza, ramen, other noodles, chewing gum, dairy products including ice cream, various soups, beverages, teas, drinks, alcoholic beverages, and vitamin complexes, all of which may be food products in the ordinary sense of the term.

Generally, in the preparation of food or beverages, the present Ponciri Fructus extract may be added in an amount of not more than 15 parts by weight, preferably not more than 10 parts by weight, per 100 parts by weight of the raw material. However, in the case of prolonged consumption for health and hygiene purposes or for health control purposes, the amount may be below above range, and the invention may also be used in the amount above the above-mentioned range, as the invention has no safety issues in terms of utilizing natural products.

The present invention also provides a composition for preventing, ameliorating or treating a muscle disease comprising auraptene as an active ingredient. The composition may be a food composition, a quasi-drug composition, or a pharmaceutical composition, as further described above.

The auraptene may be a Ponciri Fructusl-derived compound.

The muscle disease may be one or more diseases selected from the group consisting of sarcopenia, muscular atrophy, myasthenia, muscular dystrophy, sarcopenia, hypotonia, and muscular weakness, muscular dystrophy, atony, myoatrophic lateral sclerosis, and inflammatory myopathy.

The present invention also provides a composition for muscle enhancement comprising auraptene as an active ingredient. The composition may be a food composition, a quasi-drug composition, or a pharmaceutical composition, as further described above.

The composition may increase the expression of one or more genes selected from the family of endodermal cell differentiation markers comprising the genes *Myod1, Myog,* and/or *Myf5.*

The composition can increase muscle weight.

The present invention will now be described in more detail with reference to the following examples. The objects, features, and advantages of the present invention will be readily understood from the following embodiments. The invention is not limited to the embodiments described herein, but may be embodied in other forms. The embodiments described herein are provided so that the ideas of the present invention may be sufficiently conveyed to one having ordinary skill in the technical field to which the present invention belongs. Therefore, the present invention should not be limited by the following embodiments.

### [Preparation Example 1] Preparation of Ponciri Fructus Extract

Ponciri Fructus was purchased from Gyeong-dong market, cut into suitable size, and cold extracted with 6 kg of Ponciri Fructus and 10 L of 70% (v/v) ethanol in an extraction vessel, and the extract was filtered through Wattman extraction solvent filter paper. The extraction process was repeated three times, and the solvent was then concentrated under reduced pressure and dried to give 600 g of extract.

### [Preparation Example 2] Preparation of fractions of Ponciri Fructus Extract

The extract obtained according to Example 1 was subjected to solvent fractionation to obtain methylene chloride (MC), butanol (BuOH), and ethyl acetate (EA) fractions. Each fraction was prepared by suspending the 70% ethanol extract with distilled water (10 L × 3 times) and then adding an equal amount of methylene chloride (MC) to separate into a methylene chloride layer and a water layer, which was filtered and concentrated under reduced pressure to give a methylene chloride fraction (150 g). Then, the methylene chloride fraction was removed and the remaining water layer was added with an equal amount of butanol (BuOH) to obtain a butanol fraction (300 g) as described above, and the butanol fraction was removed and the remaining water layer was added with an equal amount of ethyl acetate to obtain an ethyl acetate fraction (30 g) as described above.

### [Preparation Example 3] Isolation of compounds

The compounds were isolated from the methylene chloride fraction obtained according to Example 2. Specifically, the methylene chloride fraction of Example 2 was separated by MC: MeOH= 15:1-0:15 using a silica gel open column and separated by molecular weight using a Sephadex open column under 50% methanol, and the following 13 compounds were obtained using prep-MPLC.

**Table 1**

| No. | Name of compound |
|---|---|
| 1 (Formula 1) | Melianone |
| 2 (Formula 2) | Aura ptene |
| 3 (Formula 3) | Umbelliferone |
| 4 (Formula 4) | Ponciol |
| 5 (Formula 5) | O-Methylponciol A |
| 6 (Formula 6) | O-Methylponciol B |
| 7 (Formula 7) | Triphasiol |
| 8 (Formula 8) | O-Methyltriphasiol A |
| 9 (Formula 9) | Isoponcimarin |
| 10 (Formula 10) | Triphasiolene A |
| 11 (Formula 11) | 7-(6R-hydroxy-3,7-dimethyl-2E,7-octadienyloxy) coumarin |
| 12 (Formula 12) | 7-O-(7'-peroxygeranyl)coumarin |
| 13 (Formula 13) | (E)-7-(6-hydroperoxy-3,7-dimethylocta-2,7-dienyloxy)coumarin |

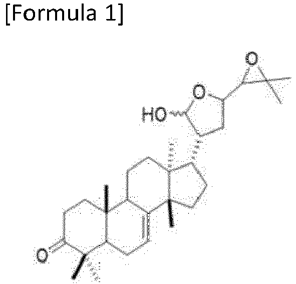

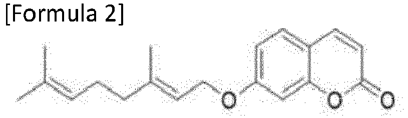

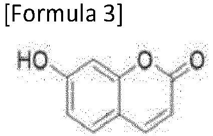

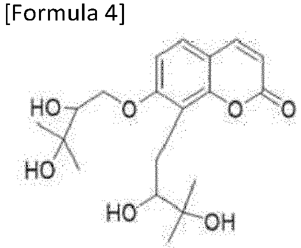

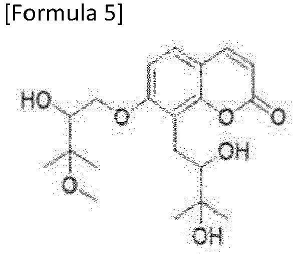

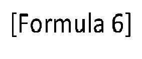

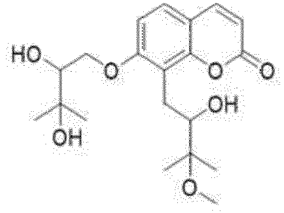

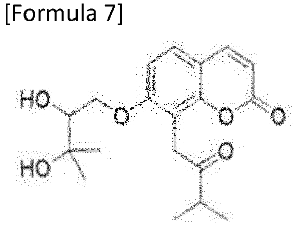

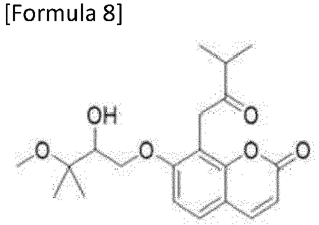

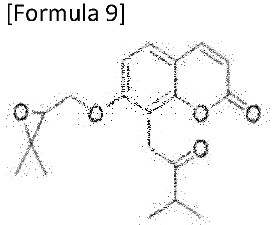

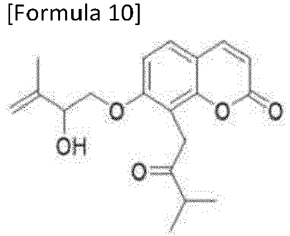

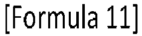

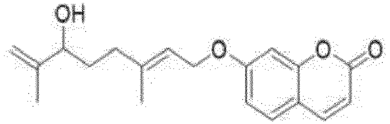

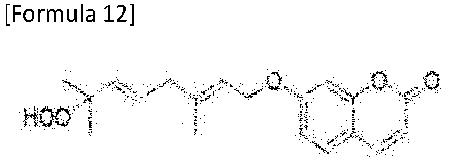

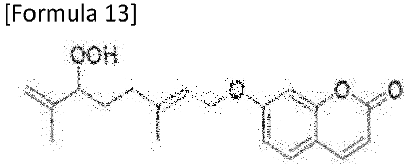

### [Example 1] Effect of Ponciri Fructus extract on improving myotube cell survival in C2C12 myotube cells in which myoatrophy was induced by dexamethasone treatment

Cell culture and differentiation of myoblasts: C2C12 mouse (CRL1772; American type cell collection, USA)-derived myoblasts were cultured in Dulbecco's Modified Eagle's Medium (DMEM) medium supplemented with 10(v/v)% FBS, and after reaching 90% confluence, they were transferred to DMEM medium containing 2(v/v)% horse serum and cultured for 7 days to differentiate into myotube cells.

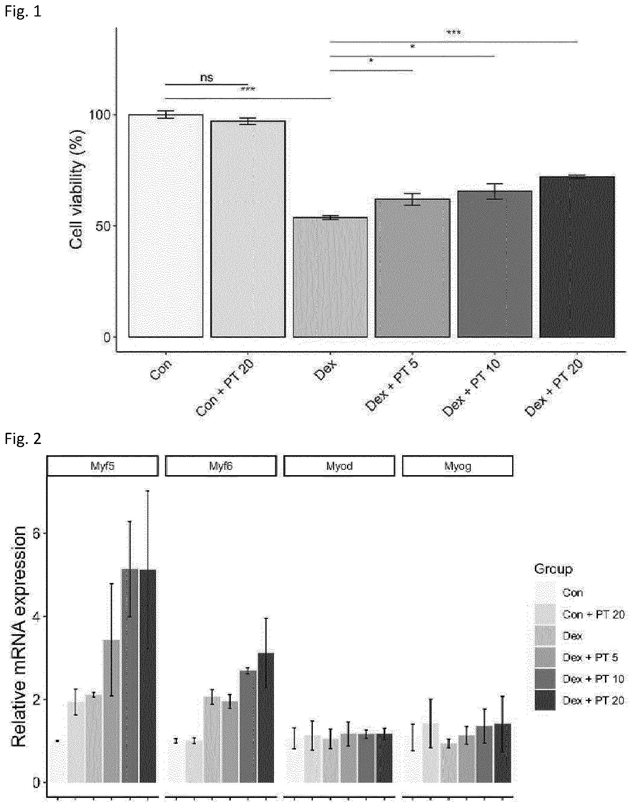

After differentiating C2C12 myoblasts into myotube cells, myoatrophy was induced by treatment with 100 µM of Dexamethasone for 24 hours, and then the myoatrophy-induced myotube cells were treated with Ponciri Fructus extract (PT) at concentrations of 5, 10, and 20 ug/ml, respectively, to determine the effect of alleviating myoatrophy. The results showed that the viability of myotube cells decreased by dexamethasone treatment was restored in the PT treatment group as shown in FIG. 1.

### [Example 2: Effect of Ponciri Fructus extract on improving myotube cell differentiation and exercise performance-related gene expression in C2C12 myotube cells in which myoatrophy was induced by dexamethasone treatment

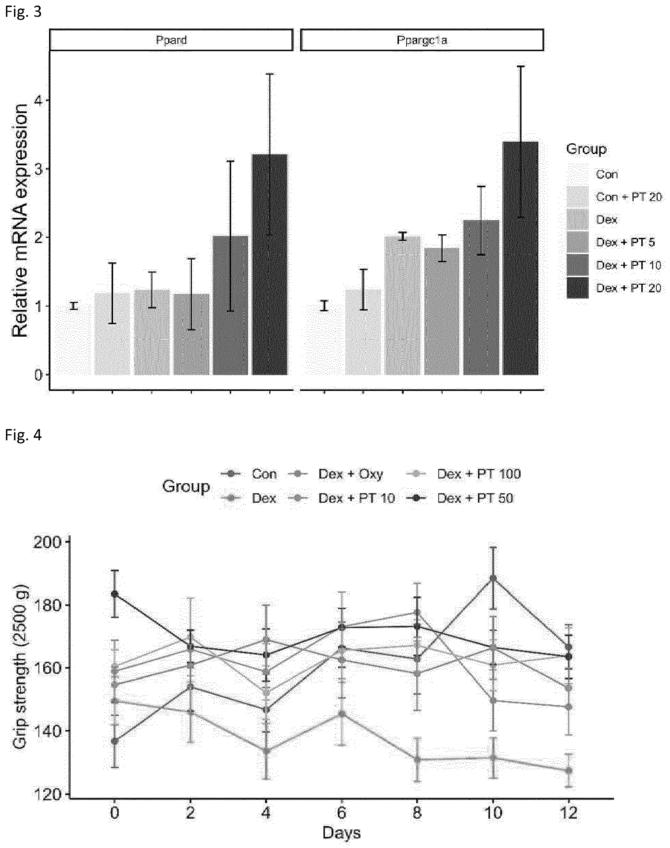

C2C12 mouse-derived endodermal cells treated with dexamethasone 10 µM were washed twice with PBS, and total RNA was extracted according to the user-recommended protocol of an RNA extraction kit (GeneAll RNA isolation kit, Seoul, South Korea). cDNA was synthesized from the extracted RNA and subjected to real time-quantitative polymerase chain reaction (RT-qPCR). PCR analysis was performed according to the user-recommended cycling conditions of analyzer (Applied Biosystems 7900 HT thermal cycler, Applied Biosystems), and to determine the expression level of each mRNA, fold change values were calculated relative to the control, using beta-actin as an endogeneous control, and relative expression amounts were compared. The primer sequences are as follows Myf5 forward primer: TCC AGG TAT TCC CAC CTG CT (sequence 1), Myf5 reverse primer: CAC CTG GAG CGG ATG AAG AAG AAC (sequence 2); Myf6 forward primer: ACA GAT CGT CGG AAA GCA GC (sequence 3), Myf6 reverse primer: CAC TCC GCA GAA TCT CCA CC (sequence 4); Myod forward primer: CAT AGA CTT GAC AGG CCC CG (sequence 5), Myod reverse primer: CGG GTC CAG GTC CTC AAA AA (sequence 6); Myog forward primer: AGC TAT CCG GTT CCA AAG CC (sequence 7), Myog reverse primer: GCA CAG GAG ACC TTG GTC AG (sequence 8); Ppard forward primer: GTA TGC GCA TGG GAC TCA CT (sequence 9), Ppard reverse primer: ACT GCC TTT ACC GTG GGT TT (SEQ ID NO: 10); Ppargcia forward primer: GTT GCC TGC ATG AGT GTG TG (SEQ ID NO: 11), Ppargcia reverse primer: CAC ATG TCC CAA GCC ATC CA (SEQ ID NO: 12). As a result, the gene expressions of Myf5, Myf6, Myod and Myog, markers of myotube cell differentiation, which were decreased by dexamethasone treatment, were increased by Ponciri Fructus extract treatment, and the gene expressions of Ppard and Pgc1a, markers of motor performance, were increased by Ponciri Fructus extract treatment, as shown in FIGS. 2 and 3.

### [Example 3] Identification of changes in muscle force in mice with dexamethasone-induced sarcopenia by Ponciri Fructus extract

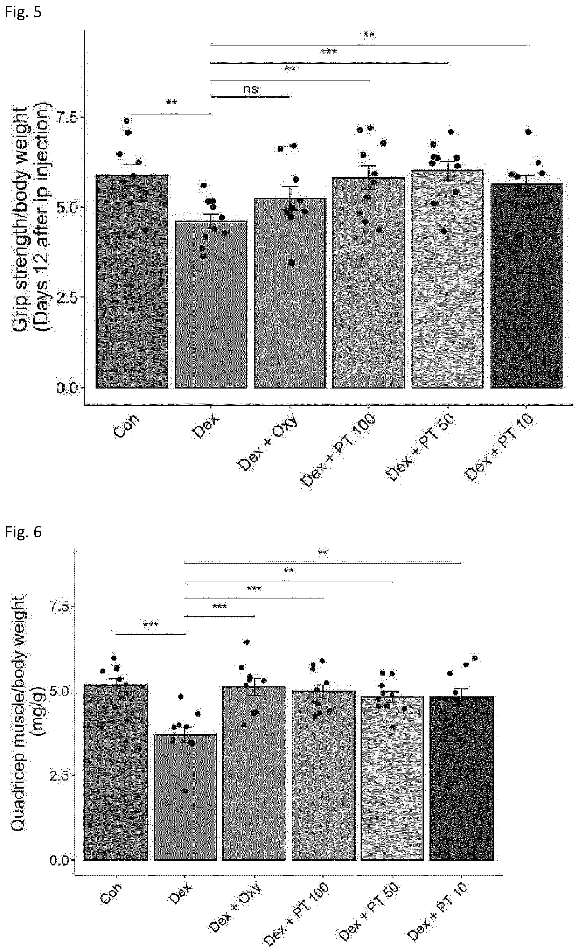

C57BL/6J mice (male, 10 weeks old) were obtained from Doo-yeol Biotech, and after 2 weeks of acclimatization, they were divided into 6 test groups (n=10 per group) according to body weight. The diets, doses, and methods of administration to the experimental groups are shown in the table below. The test substance was prepared as a liquid suspension using 0.5% CMC, and the test substance was administered for 4 weeks. To compensate for the placebo effect of 0.5% CMC and the weight loss effect caused by the stress of administration, the vehicle control group was administered 0.5% CMC orally daily. Two weeks after treatment, the experimental group received an intraperitoneal injection of dexamethasone for 2 weeks to induce sarcopenia, while the normal control group received an intraperitoneal injection of saline. All mice were fed the same AIN76 diet (Research Diets) and treated with the test compound for an additional 2 weeks for a total of 4 weeks. The dark : light cycle was maintained at a 12 hr : 12 hr interval and water was allowed ad libitum. As a result, it was found that the muscle force decreased in the Dexamethasone (Dex) group over time as shown in FIG. 4 and FIG. 5, and the muscle force increased in the group of mice fed with the positive control Oxymetholone and Ponciri Fructus extract compared to the group treated with Dexamethasone alone.

**[Table 2]**

| Test group | Fodder | Sarcopenia Inducing Substances | Orally Administered Substances | Dosage and method of administrati on | Duration of administration |
|---|---|---|---|---|---|
| 1(Con) | AIN76 diet | Saline | Vehicle | - | After oral administration of test substance for 2 weeks, administration of test substance and Inducing Substance for 2 weeks (Total 4 weeks) |
| 2 (Dex) | | Dexamethasone (20 mg/kg, once daily, 2 weeks) | Vehicle | - | |
| 3 (Dex+Oxy) | | | Oxymetholone | 50 mg/kg, once daily, | |
| 4 (Dex+ PT 10) | | | Ponciri Fructus Extract | 10 mg/kg, once daily | |
| 5 (Dex+ PT 50) | | | | 50 mg/kg, once daily | |
| 6 (Dex+ PT 100) | | | | 100 mg/kg, once daily | |

### [Example 4] Determination of weight gain by muscle types in dexamethasone-induced sarcopenia in mice treated with Ponciri Fructus extract

At the end of a total of 4 weeks of Ponciri Fructus extract administration in Example 3 above, the weight of each type of muscle tissue in all treatment groups was determined.

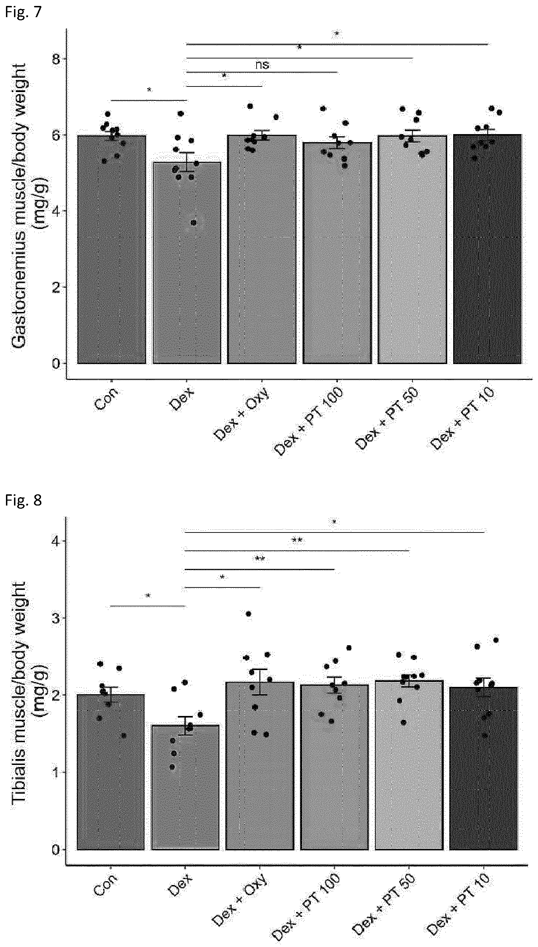

As a result, as shown in FIGS. 6 to 8, the weight of quadriceps muscle, gastrocnemius muscle, and tibialis muscle was decreased in the Dexamethasone treated group compared to the saline treated group, and the weight of these muscles was significantly increased by administration of the positive control Oxymetholone and Ponciri Fructus extract.

### [Example 5] Confirmation of the efficacy of Ponciri Fructus extract in improving exercise performance in mice with dexamethasone-induced sarcopenia

At the end of a total of 4 weeks of Ponciri Fructus extract administration in Example 3 above, Distance to exhaustion, maximal speed capacity, and time to exhaustion for all treatment groups was determined.

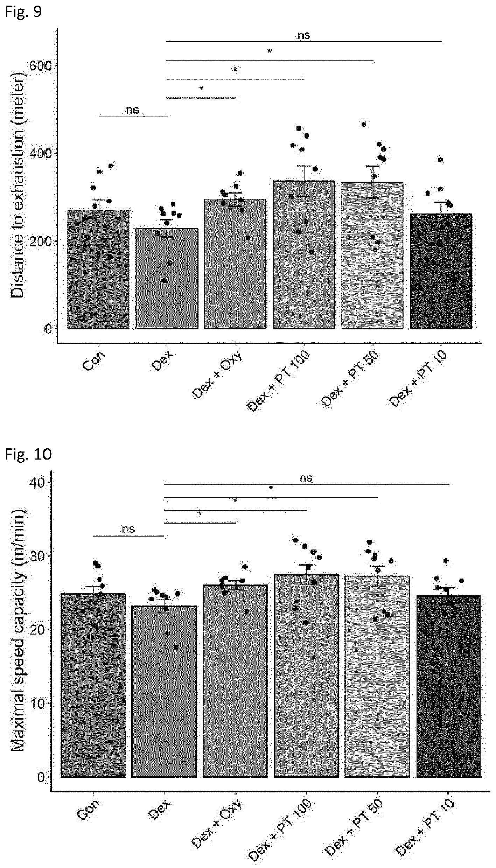

As a result, as shown in FIGS. 9 to 11, there was a significant increase in exercise performance with administration of Ponciri Fructus extract.

### [Example 6] Cell viability assay by fractions

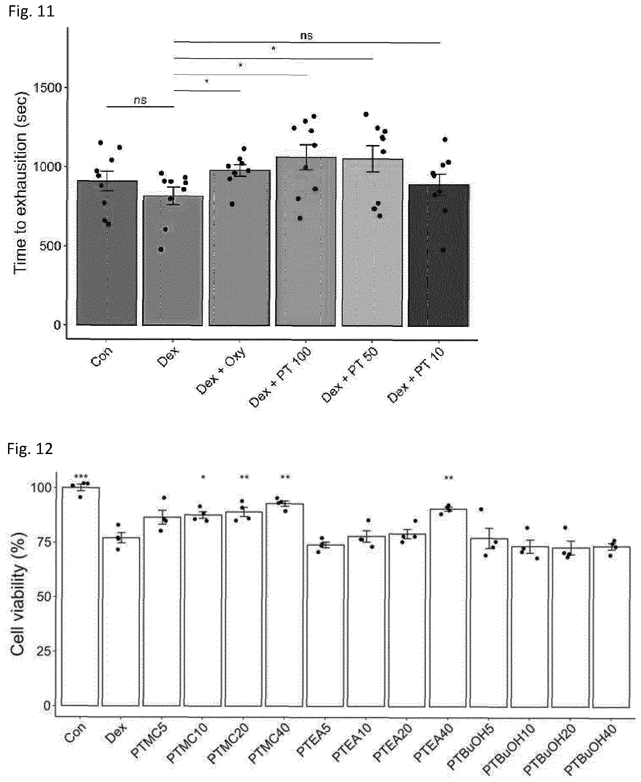

The effect of each fraction (concentrations 5 ug/ml, 10 ug/ml, 20 ug/ml, 40 ug/ml) prepared according to Example 2 on the viability of endodermal cells in which myoatrophy was induced by dexamethasone was analyzed. The assay was performed in the same manner as in Example 1. As shown in FIG. 12, it was found that the methylene chloride fraction best restored the viability of myotube cells reduced by dexamethasone treatment.

### [Example 7] Cell viability assay by Ponciri Fructus-derived compounds

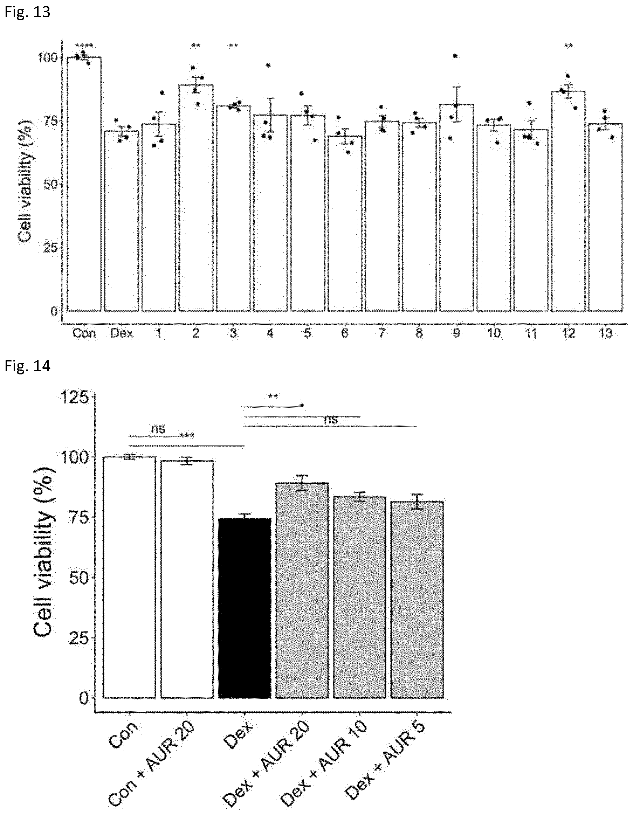

The effect of the compounds isolated according to Example 3 (each at a concentration of 20 uM) on the viability of myotube cells in which myoatrophy was induced by dexamethasone was analyzed. The assay was performed in the same manner as in Example 1. As shown in FIG. 13, most of the compounds were found to restore the viability of myotube cells reduced by dexamethasone treatment, and in particular, compounds 2, 3, 9, and 12 were found to excellently restore the viability of myotube cells reduced by dexamethasone treatment.

### [Example 8] Efficacy analysis of auraptene

### Example 8-1. Analysis of Cell Viability

Cell viability was assayed by concentration for auraptene (compound 2), the compound that showed the best effect in Example 7 above. The assay was performed in the same manner as in Example 1. As shown in FIG. 14, auraptene was found to restore the viability of myotube cells reduced by dexamethasone treatment in all experimental groups at 5 uM, 10 uM, and 20 uM, respectively.

### Example 8-2. Diameter analysis of myotube cells

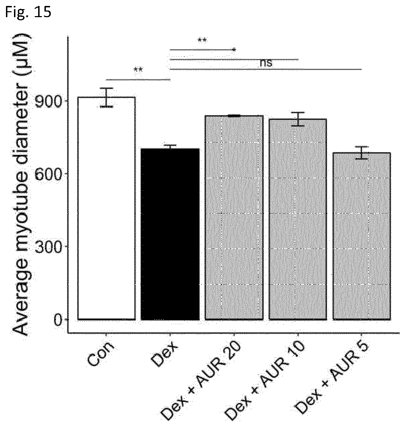

The effect of auraptene (5 uM, 10 uM, 20 uM) on the diameter of myotube cells in which myoatrophy was induced by dexamethasone (100 µM) was analyzed. The results confirmed that the diameter of myotube cells decreased by dexamethasone treatment was restored by auraptene treatment (FIG. 15).

### Example 8-3. Effect of enhancing gene expression related to myotube cell differentiation and exercise performance

The effect of auraptene on promoting myotube cell differentiation and exercise performance-related gene expression in C2C12 myotube cells with dexamethasone-induced myoatrophy was analyzed. The assay was performed using the same method as in Example 2. The primer sequences were as follows:
Myod1 forward primer: CAT AGA CTT GAC AGG CCC CG (SEQ ID NO: 5),
Myod1 reverse primer: CGG GTC CAG GTC CTC AAA AA (SEQ ID NO: 6),
Myog forward primer: AGC TAT CCG GTT CCA AAG CC (SEQ ID NO: 7),
Myog reverse primer: GCA CAG GAG ACC TTG GTC AG (SEQ ID NO: 8),
Myf5 forward primer: TCC AGG TAT TCC CAC CTG CT (SEQ ID NO: 1),
Myf5 reverse primer: CAC CTG GAG CGG ATG AAG AAG AAC (SEQ ID NO: 2),
Pparg1a forward primer: GTT GCC TGC ATG AGT GTG TG (SEQ ID NO: 11),
Pparg1a reverse primer: CAC ATG TCC CAA GCC ATC CA (SEQ ID NO: 12),
Ucp3 forward primer: GTT TTG CGG ACC TCC TCA CT (SEQ ID NO: 13),
Ucp3 reverse primer: CTC TGT GCG CAC CAT AGT CA (SEQ ID NO: 14),
Nrf1 forward primer: CCC GTG TTC CTT TGT GGT GA (SEQ ID NO: 15),
Nrf1 reverse primer: ATT CCA TGC TCT GCT GCT GG (SEQ ID NO: 16),
Tomm20 forward primer: TGT GCG GTG TGT TGT CTG TT (SEQ ID NO: 17),
Tomm20 reverse primer: TAA GTG CCC AGA GCA CAG GA (SEQ ID NO: 18).

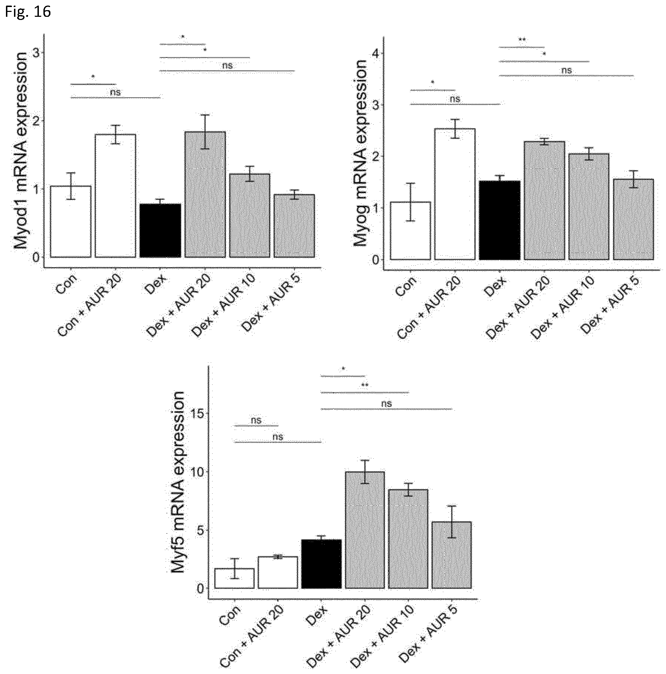

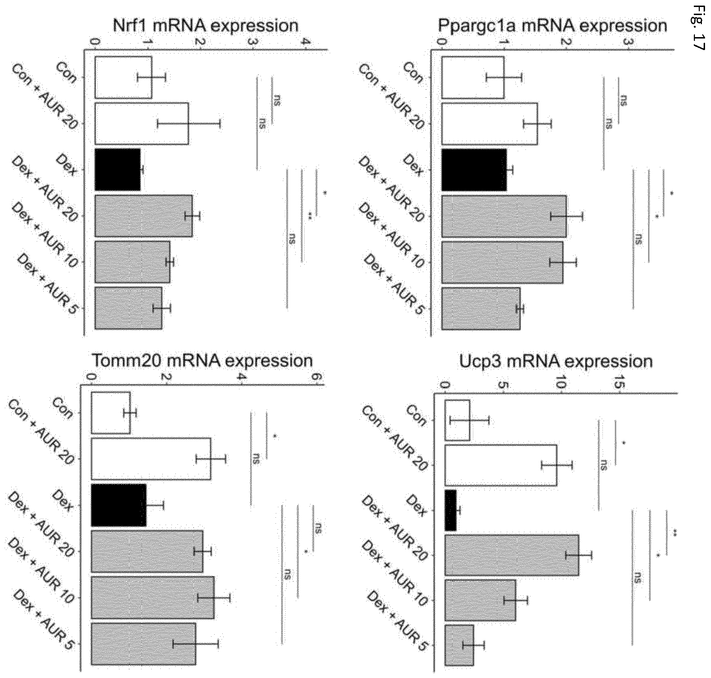

As shown in FIGS. 16 and 17, gene expression of the myotube cell differentiation markers, *Myod1, Myog,* and Myf5, which were decreased by dexamethasone treatment, and the motor performance differentiation markers, *Pparg1a, UCP3, Nrf1,* and *Tomm20,* were found to be increased by auraptene treatment (FIG. 16, FIG. 17).

### Example 8-4. Efficacy in improving oxidative stress and mitochondrial function

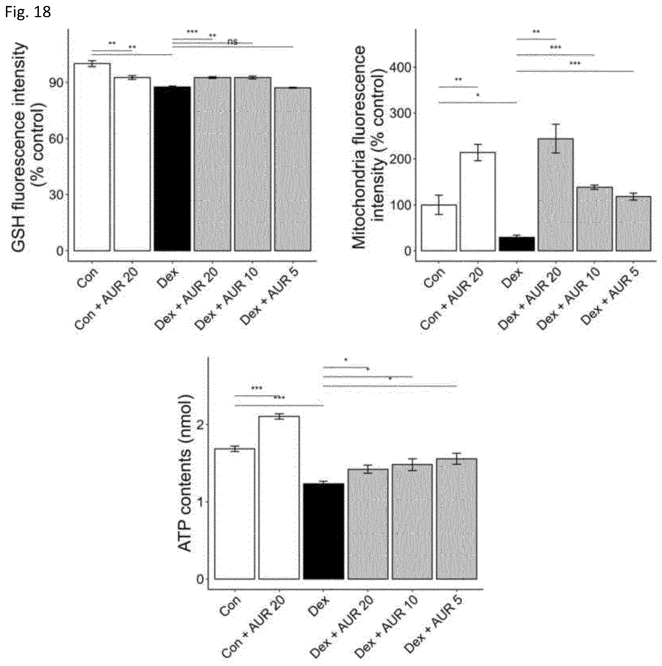

C2C12 myoblasts were differentiated into myotube cells as in Example 1, and then treated with 10 µM dexamethasone for 24 hours to induce oxidative stress or mitochondrial dysfunction, and then treated with auraptene to determine its efficacy in improving function. Dexamethasone treatment decreased glutathione (GSH) content, which has antioxidant activity, but auraptene treatment increased GSH content again, and mitochondrial dysfunction induced by dexamethasone treatment was improved by auraptene treatment (FIG. 18).

### Example 8-5. Determine changes in muscle force in mice with dexamethasone-induced sarcopenia

The changes in muscle force in mice were determined by the same method as in Example 3 above. The diets, doses, and methods of administration of the experimental groups are shown in the table below.

**[Table 3]**

| Test group | Fodder | Sarcopenia Inducing Substances | Orally Administered Substances | Dosage and method of administrati on | Duration of administration |
|---|---|---|---|---|---|
| Con | AIN76 diet | Saline | Vehicle | - | After oral administration of test substance for 2 weeks, administration of test substance and Inducing Substance for 2 weeks (Total 4 weeks) |
| Dex | | Dexamethasone (20 mg/kg, once daily, 2 weeks) | Vehicle | - | |
| Dex + AUR50 | | | auraptene | 50 mg/kg, once daily | |
| Dex + AUR 10 | | | | 10 mg/kg, once daily | |

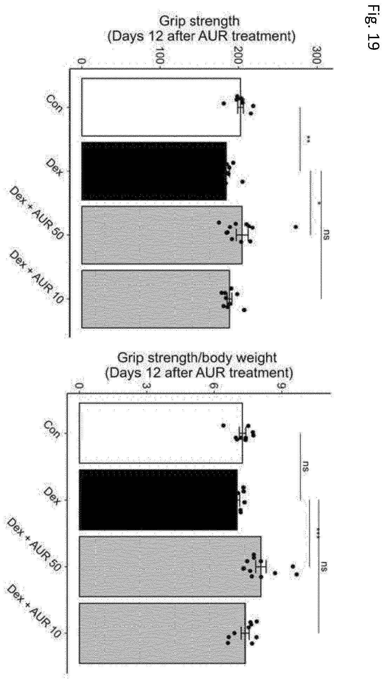

As shown in FIG. 19, we found that the muscle force decreased over time in the dexamethasone (Dex)-treated group and increased in the auraptene-fed mouse group compared to the dexamethasone-treated group.

### Example 8-6. Determining weight gain by muscle types in mice with dexamethasone-induced sarcopenia

After a total of 4 weeks of Auraptene administration, the weight of each type of muscle tissue in all treatment groups was determined in Example 8-5 above.

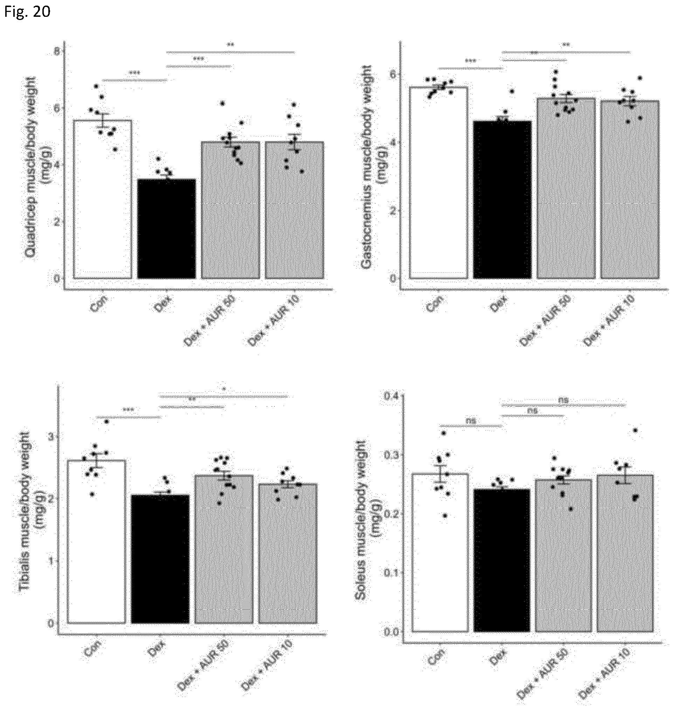

As a result, as shown in FIG. 20, compared to the saline-treated control group, the dexamethasone-treated group was found to have decreased thigh muscle, longissimus dorsi muscle, calf muscle, and soleus muscle weights, but the treatment of auraptene significantly increased these muscle weights.

## Claims

1. A pharmaceutical composition for use in prevention or treatment of a muscle disease, comprising Ponciri Fructus extract or a fraction thereof as an active ingredient.

2. The pharmaceutical composition for use of claim 1, wherein the muscle disease is one or more diseases selected from the group consisting of sarcopenia, muscular atrophy, myasthenia, muscular dystrophy, sarcopenia, hypotonia, and muscular weakness, muscular dystrophy, atony, myoatrophic lateral sclerosis, and inflammatory myopathy.

3. The pharmaceutical composition for use of claim 1, wherein the composition increases expression of one or more genes selected from the family of myotube cell differentiation markers comprising the genes Myf5, Myf6, Myod, and/or Myog.

4. The pharmaceutical composition for use of claim 1, wherein the composition increases expression of the gene Ppard or Pgc1a, which are markers of athletic performance.

5. The pharmaceutical composition for use of claim 1, wherein the composition is extracted with a solvent selected from the group consisting of water, organic solvents, and mixtures thereof.

6. The pharmaceutical composition for use of claim 5, wherein the organic solvent is one or more solvents selected from the group consisting of lower alcohols of carbon number 1 to 6, nucleic acids, acetone, ethyl acetate, chloroform, and diethyl ether.

7. A food composition for use in the prevention or amelioration of a muscle disease, comprising Ponciri Fructus extract or a fraction thereof as an active ingredient.

8. The food composition for use of claim 7, wherein the muscle disease is one or more diseases selected from the group consisting of sarcopenia, muscular atrophy, myasthenia, muscular dystrophy, sarcopenia, hypotonia, and muscular weakness, muscular dystrophy, atony, myoatrophic lateral sclerosis, and inflammatory myopathy.

9. The food composition for use of claim 7, wherein the composition increases expression of one or more genes selected from the family of myotube cell differentiation markers comprising the genes Myf5, Myf6, Myod, and/or Myog.

10. The food composition for use of claim 7, wherein the composition increases expression of the gene Ppard or Pgc1a, which are markers of athletic performance.

11. The food composition for use of claim 7, wherein the composition is extracted with a solvent selected from the group consisting of water, organic solvents, and mixtures thereof.

12. The food composition for use of claim 11, wherein the organic solvent is one or more solvents selected from the group consisting of lower alcohols of carbon number 1 to 6, nucleic acids, acetone, ethyl acetate, chloroform, and diethyl ether.

13. A quasi-drug composition for use in the prevention or amelioration of a muscle disease, comprising Ponciri Fructus extract or a fraction thereof as an active ingredient.
